# EUROPEAN PATENT SPECIFICATION

(11) **EP 3 407 904 B1**
(45) Date of publication and mention of the grant of the patent: **25.08.2021**
(21) Application number: 17707095.0
(22) Date of filing: 27.01.2017
(51) Int. Cl.: A61K 36/537, A61P 25/28, A61P 19/10, A61P 21/00, A61P 35/00, A61P 43/00

(54) **BOTANICAL COMPOSITIONS OF SALVIA OFFICINALIS WITH ACTIVITY IN EXTENDING CHRONOLOGICAL LIFESPAN AND USES THEREOF**
PFLANZLICHE ZUSAMMENSETZUNGEN VON SALVIA OFFICINALIS MIT AKTIVITÄT ZUR VERLÄNGERUNG DER CHRONOLOGISCHEN LEBENSERWARTUNG UND DEREN VERWENDUNG
COMPOSITIONS BOTANIQUES DE SALVIA OFFICINALIS À ACTIVITÉ PROLONGEANT LA DURÉE DE VIE CHRONOLOGIQUE ET LEUR UTILISATION

(30) Priority: 27.01.2016 GB 201601520
(43) Date of publication of application: 05.12.2018
(73) Proprietor: Sibelius Limited, Milton Park Abingdon Oxfordshire OX14 4SA (GB)
(72) Inventor: LEYLAND, Peter Anthony, Oxford OX1 2NP (GB); AKOULITCHEV, Alexandre, Oxford OX1 2NP (GB); VERSLYPE, Antoine, Oxford OX1 2NP (GB); CAIGER, Stephen, Oxford OX1 2NP (GB)
(74) Representative: Crease, Devanand John
(86) International application number: PCT/GB2017/050209
(87) International publication number: WO 2017/129987

(56) References cited:
- WO-A1-02/13840
- US-A1- 2012 282 360
- JASSBI AMIR REZA ET AL: "Bioactive phytochemicals from shoots and roots ofSalviaspecies", PHYTOCHEMISTRY REVIEWS, KLUWER, NL, vol. 15, no. 5, 1 August 2015 (2015-08-01) , pages 829-867, XP036065244, ISSN: 1568-7767, DOI: 10.1007/S11101-015-9427-Z [retrieved on 2015-08-01]
- TOMASZ DZIURZYNSKI ET AL: "Biological activities of Salvia L species", CURRENT ISSUES IN PHARMACY AND MEDICAL SCIENCES, vol. 26, no. 3, 30 September 2013 (2013-09-30), pages 326-330, XP055362978, PL ISSN: 2084-980X, DOI: 10.12923/j.2084-980X/26.3/a.19
- NIGEL B. PERRY ET AL: "Essential Oils from Dalmatian Sage ( Salvia officinalis L.): Variations among Individuals, Plant Parts, Seasons, and Sites", JOURNAL OF AGRICULTURAL AND FOOD CHEMISTRY, vol. 47, no. 5, 1 May 1999 (1999-05-01), pages 2048-2054, XP055362886, US ISSN: 0021-8561, DOI: 10.1021/jf981170m
- PINTO ET AL: "In vitro susceptibility of some species of yeasts and filamentous fungi to essential oils of Salvia officinalis", INDUSTRIAL CROPS AND PRODUCTS, ELSEVIER, NL, vol. 26, no. 2, 12 June 2007 (2007-06-12), pages 135-141, XP022113931, ISSN: 0926-6690, DOI: 10.1016/J.INDCROP.2007.02.004
- JAMISON ET AL.: "Sage (Salvia Officinalis) ameliorates experimentally induced neurodegeneration in C. elegans", FASEB JOURNAL, vol. 26, no. 1, 2012, page 1025, SAN DIEGO , CA , USA
- TILDESLEY ET AL.: "Positive modulation of mood and cognitive performance following administration of acute doses of Salvia lavandulaefolia essential oil to healthy young volunteers", PHYSIOLOGY AND BEHAVIOR, vol. 83, no. 5, 2005, pages 699-709,

## Description

### FIELD

The invention relates to compositions comprising extracts of botanical origin, most particularly from sage species *Salvia officinalis,* which extracts have defined biological activity.

### BACKGROUND OF THE INVENTION

*Salvia officinalis* has been widely used in commerce and medicine for centuries, providing broad areas of activity and a seemingly exceptional safety profile. As a herb and a herbal medicine there is a long history of human experience with *S. officinalis.* Among the Salvia genus the common sage, species *S. officinalis* is one of the oldest cultivated and most widely used herbs for medicinal, culinary, cosmetic, sensory and other purposes with recorded applications dating back to the 1st century CE (Pliny). *S. officinalis* was in common usage throughout Europe by medieval times, and features in British herbal apothecaries from the 16th century onwards (Crellin, J.K. and Philpot, J. (1990). Herbal Medicine Past and Present. Duke University Press, Durham, U.K. 374-376). Its suggested uses included those as a general treatment to enhance 'head and brain' functioning, improve the memory, and quicken the senses. The many contemporary indications for *S. officinalis* include the alleviation of poor memory, mental confusion, depression, vertigo, as an anti-inflammatory, and use as a treatment for the symptoms of the menopause (Bartram, T. (1995). Encyclopaedia of Herbal Medicine. 1st Ed. Grace Publishers, London, U.K. pp: 379.; British Herbal Pharmacopeia, 1983). Dried extract of *S. officinalis* has been shown to improved cognitive performance in human subjects on a number of tasks mainly contributing to the formation of secondary memory (Scholey (2008) Psychopharmacology (Berl). May;198(1):127-39).

In terms of the whole herb, both sage species contain about 1.0-2.8 % volatile oil (Leung AY, Foster S. (1996) Encyclopaedia of common natural ingredients. Chichester: Wiley; 1996), and it has been suggested that the monoterpenoid constituents (a-pinene, β-pinene, 1,8-cineole, thujone, camphor, geraniol), comprise the active components of the whole herb (Perry et al. (2001) J Pharm Pharmacol. Oct;53(10):1347-56). The European Pharmacopoeia describes sage leaf (*S*. *officinalis*) as particularly rich in thujone and a study by Walch et al. established levels of thujone in dried food preparations of sage at between 944 to 1353 mg/kg and a camphor content of 1651 to 4322 mg/kg (Chemistry Central Journal (2011) 5:44). Indeed, thujone is considered to be one of the key active ingredients responsible for the purported therapeutic and nutraceutic properties of sage-based products. *Salvia lavandulaefolia* (Spanish Sage) has a similar composition to S. officinalis, with the exception that it lacks a high concentration of thujone, which can be toxic in large doses. It has therefore been suggested that *S. lavandulaefolia* may provide an efficacious, but more theoretically suitable, treatment (Mantle et al. (2000), CNS Drugs;13:201- 13).

Sage is marketed worldwide in numerous preparations and products for human nutrition, food flavouring and preservation, medicinal and cosmetic use. Globally sage products and preparations are used by literally millions of people for culinary use, in natural herbal medicines, in cosmetics, in fragrances, and personal hygiene products.

Several molecules and signalling pathways have been analysed by clinical researchers in relation to the controlling mechanism of lifespan and metabolism (e.g. Sirtuins, Resveratrol, Rapamycin, Insulin Signalling and TOR) and the pharmaceutical industry has been quick to recognise those opportunities. Rapamycin, for example, extends the life of elderly mice by 9-14%. Rapamycin (acting via TORC1) and can extend chronological lifespan in yeast by 230%. Currently, rapamycin is the gold standard for life-extension drugs.

Resveratrol is a natural product derived from the skin of grapes, blueberries, raspberries, and mulberries and is able to increase lifespan in yeast, as well as to improve health in animal models of diseases of ageing. Most recent research suggests that resveratrol is active in the PARP1 pathway and contributes to the initiation of a stress response that induces survival expression of genes (Sajish & Schimmel (2015) Nature Mar 19; 519(7543): 370-3).

AR Jassbi et al: "Bioactive phytochemicals from shoots and roots of Salvia species", Phytochemistry Reviews, vol. 15, no. 5, 1 August 2015, discusses phytochemical analyses of various sage plants. Diverse biologically active phytochemicals in the sage plants are discussed and their various cytotoxic and antimicrobial, antiprotozoal, antioxidant, phytotoxic and insecticide effects, as well as their candidacy for the formulation of natural medicines, are considered.

US 2012/282360 A1 describes herbal preparations comprising sage, thyme, and cumin materials in effective amounts for relieving symptoms of inflammation resulting from PGE₂, TNFα, COX-2 and COX-1.

In view of the widespread cultural acceptance of sage-based natural products within society it would be advantageous to provide compositions with altered or enhanced nutraceutical and therapeutic properties. In particular, it would be desirable to identify sage-based products having proven nutritional specificity for defined conditions or pathological states, particularly those relating to controlling lifespan, age-related pathologies and metabolism.

The compositions and methods of the present invention address that above shortcomings in the art. These and other uses, features and advantages of the invention should be apparent to those skilled in the art from the teachings provided herein.

### SUMMARY OF THE INVENTION

In accordance with a first aspect, the invention provides a composition comprising a nutritionally or pharmaceutically effective dosage of an extract of *Salvia officinalis* and an acceptable carrier for use in the treatment of age-related pathologies in a human or animal subject, wherein the age-related pathologies are selected from one or more of the group consisting of: cancer; neurodegenerative disease, including Alzheimer's Disease and Parkinson's Disease; cognitive decline; osteoporosis; and sarcopenia; and wherein the extract is obtained from the leaf of a variety of *Salvia officinalis* and has a thujone content of no more than around 1ppm. Suitably the thujone content is less than around 1 ppm. Optionally the variety/cultivar of *Salvia officinalis* also has a low camphor content in the leaf, as defined by a camphor content of less than 25 ppm, suitably not more than 10 ppm, optionally at most 7 ppm.

The extract may be obtained from the leaf of the *Salvia officinalis,* suitably the extract is obtained from the dried leaf of the *Salvia officinalis.*

The cognitive decline may comprise age-related memory impairment. The subject may be a human (optionally an adult, geriatric or paediatric patient). Alternatively, the subject may be a non-human animal.

The composition may be intended for oral administration, suitably administered in the form of a tablet or capsule. The composition may comprise at least 1%, optionally at least 3%, by weight of the extract of *Salvia officinalis.* The composition may comprise no more than 85% by weight of the extract of *Salvia officinalis,* suitably wherein the composition comprises no more than 40% by weight of the extract of *Salvia officinalis.*

The composition may be administered to the subject daily or more than once daily.

In specific embodiments of the invention the composition is administered in an amount of not less than about 1 mg of the active component, typically not less than about 10 mg of the active component, optionally not less than about 50 mg of the active component, suitably not less than about 100 mg of the active component. Suitably the composition is administered in an amount of not more than about 25g of the active component, suitably not more than about 10g, and optionally not more than about 1g.

In certain embodiments the composition is capable of eliciting an increase in median lifespan in yeast cells of at least 5% suitably at least 7.5%, optionally at least 10% when compared to yeast cells that have not been exposed to the composition. The composition may further or alternatively be capable of eliciting an increase in mean lifespan in *C. elegans* worms of at least 10%, suitably at least 12%, optionally at least 20% when compared to worms that have not been exposed to the composition.

Also described herein is a method for increasing the chronological lifespan of cells within the body of a subject comprising administering a nutritionally or pharmaceutically effective dosage of an extract of *Salvia officinalis* to the subject, wherein the extract is obtained from a variety of *Salvia officinalis* that has a low thujone content in the leaf. Suitably the subject is a human (optionally an adult, geriatric or paediatric patient). Alternatively, the subject may be a non-human animal.

The method may be a cosmetic method of treatment or a therapeutic method of treatment

In some arrangements the composition elicits an increase in mean lifespan in *C. elegans* worms of at least 10%, suitably at least 12%, optionally at least 20% when compared to worms that have not been exposed to the composition.

In one arrangement the cells are comprised within the central nervous system (CNS) of the subject.

Also described herein is a method for treating age-related pathologies in a within the body of a human or animal subject comprising administering a nutritionally or pharmaceutically effective dosage of an extract of *Salvia officinalis* to the subject, wherein the extract is obtained from a variety of *Salvia officinalis* that has a low thujone content in the leaf, and wherein the age-related pathologies are selected from one or more of the group consisting of: cancer; neurodegenerative disease, including Alzheimer's Disease and Parkinson's Disease; cognitive decline; osteoporosis; and sarcopenia. Typically the method comprises administering a composition as described herein to the subject.

Also described herein is a method for identifying whether an extract of a cultivar of *Salvia officinalis* demonstrates efficacy as a treatment of an age-related pathology in a human or animal subject, the method comprising exposing a population of *C. elegans* worms to a composition that comprises the extract and determining whether the composition elicits an increase in mean lifespan in the population of at least 10%, suitably at least 12%, optionally at least 20% when compared to a population that has not been exposed to the composition.

Also provided for is a composition identified by the method described immediately above, for use in the treatment of one or more age-related disease selected from the group consisting of: cancer; neurodegenerative disease, including Alzheimer's Disease and Parkinson's Disease; cognitive decline; osteoporosis; and sarcopenia.

### BRIEF DESCRIPTION OF THE DRAWINGS

The invention is further exemplified in the accompanying drawings in which:
Figure 1 is a plot showing comparison between target raw material from *S. officinalis* (TRM) - surrounded by dashed line - and non-target species (NTS) following multivariate analysis of NMR spectra of leaf extract samples.
Figure 2 is a representation of an outliers diagnostic chart further comparing TRM (surrounded by dashed line) and NTS.
Figure 3 is a plot showing comparison of TRM (surrounded by dashed line) with samples of different varieties obtained from the UK (bottom right), Croatia (bottom left) and Italy (top right).
Figure 4 is a representation of an outliers diagnostic chart further comparing TRM (surrounded by dashed line) and the samples of Figure 3.
Figure 5 is a plot showing comparison of TRM (surrounded by dashed line) with samples obtained out of season.
Figure 6 is a representation of an outliers diagnostic chart further comparing TRM (surrounded by dashed line) and the samples of Figure 5.
Figure 7 is bar chart showing the effects of N92 extract treatment on yeast median lifespan.
Figure 8 is a graph that shows the survival profiles for nematode worms exposed to DMSO control (boxes) versus treatment with N92 sage extract at 10mg/ml (diamonds).
Figure 9 is a graph that shows the survival profiles for nematode worms exposed to DMSO control (boxes) versus treatment with N92 sage extract at three different concentrations 0.1mg/ml (line), 1 mg/ml (stars) and 10 mg/ml (diamonds).
Figure 10 are graphs that show the survival profiles for nematode worms exposed to DMSO control (boxes) versus treatment with (a) red sage (N315), (b) an English sage (N316) and (c) a Spanish sage (N318), all tested at a concentration of 10mg/ml.
Figure 11 are graphs that show the survival profiles for nematode worms exposed to DMSO control (boxes) versus treatment with (a) donepezil (N350) at 10mg/ml, (b) rivastigmine (N355) at 10mg.ml, (c) galantamine (N354) at 10mg/ml, and (d) *S. officinalis* sage UK source A (N314) at 10mg/ml.
Figure 12 is a bar chart showing the extension of mean chronological life span in *C. elegans* of small molecules and two distinct sage extracts at dosages of 10mg/ml. The increase is the percentage increase versus untreated control.

### DETAILED DESCRIPTION OF THE INVENTION

Unless otherwise indicated, the practice of the present invention employs conventional techniques of chemistry, molecular biology, microbiology, recombinant DNA technology, and chemical methods, which are within the capabilities of a person of ordinary skill in the art. Such techniques are also explained in the literature, for example, M.R. Green, J. Sambrook, 2012, Molecular Cloning: A Laboratory Manual, Fourth Edition, Books 1-3, Cold Spring Harbor Laboratory Press, Cold Spring Harbor, NY; Ausubel, F. M. et al. (1995 and periodic supplements; Current Protocols in Molecular Biology, ch. 9, 13, and 16, John Wiley & Sons, New York, N. Y.); B. Roe, J. Crabtree, and A. Kahn, 1996, DNA Isolation and Sequencing: Essential Techniques, John Wiley & Sons; J. M. Polak and James O'D. McGee, 1990, In Situ Hybridisation: Principles and Practice, Oxford University Press; M. J. Gait (Editor), 1984, Oligonucleotide Synthesis: A Practical Approach, IRL Press; and D. M. J. Lilley and J. E. Dahlberg, 1992, Methods of Enzymology: DNA Structure Part A: Synthesis and Physical Analysis of DNA Methods in Enzymology, Academic Press.

Prior to setting forth the invention, a number of definitions are provided that will assist in the understanding of the invention. Unless otherwise defined, all technical and scientific terms used herein have the same meaning as commonly understood by one of ordinary skill in the art to which this invention belongs.

As used herein, the term "comprising" means any of the recited elements are necessarily included and other elements may optionally be included as well. "Consisting essentially of' means any recited elements are necessarily included, elements that would materially affect the basic and novel characteristics of the listed elements are excluded, and other elements may optionally be included. "Consisting of" means that all elements other than those listed are excluded. Embodiments defined by each of these terms are within the scope of this invention.

The present invention is based in part upon the identification and characterisation of unexpected and unique properties associated with a composition derived from particular varieties of *S. officinalis.*

The present invention resides in the provision of a formulation that may be taken as a nutritional supplement, or "nutraceutical", or as a pharmaceutical composition that is able to contribute to an increase in cellular chronological lifespan in an individual. In particular, the compositions and formulations of the present invention are able to arrest ageing-related pathologies, including cognitive decline and neurodegeneration. The formulation of the invention comprises unique complex of botanically derived components that surprisingly act in synergy to achieve the above-mentioned effects.

The term "nutritional supplement" is used herein to denote a composition that is directed for recipient human and/or animal consumption and which is intended to provide additive nutritional benefit above and beyond the existing diet of the recipient. The nutritional benefits of the supplement may include biological activities that are essentially pharmacological, although, typically nutritional supplements do not exhibit the severity of side effects associated with pharmaceutical compositions.

The term "active component" is used herein to denote specified components or ingredients that are present within the compositions of the invention and which contribute to the desired activity of said compositions. The active components of the invention may be isolated compounds, or may comprise specified extracts of botanical origin. It is desirable for the active components of the invention to be standardised, that is to say provided at a specific concentration of known activity. According to the compositions of the invention the mass of active component is given in milligrams (mg) and is equivalent to the mass of compound within the composition that possesses the desired biological activity. By way of example, a composition of the invention that comprises 250 mg of *S. officinalis* extract per dose is considered as comprising 250 mg of the essential combination of compounds that contribute to and define the required biological activity of the S. *officinalis* extract of the invention. It will be appreciated that an extract of botanical origin is likely to comprise many thousands of different potential active compounds and combinations. In a specific embodiment of the invention the active component is comprised within a dried ethanolic extract of *S. officinalis* leaf material obtained from a cultivar having the desired chemical properties.

In terms of the whole herb, *S. officinalis* cultivars typically contain about 1.0 - 2.8% volatile oil. Extracts contain a number of polyphenolic compounds, most notably rosmarinic acid, methyl carnosate, caffeic acid, luteolin 7-0-glucoside, and luteolin and they also tend to contain alpha- and beta-thujone as a major component (usually around 50% of volatile oil). In traditional sage herbal products, the volatile monoterpenoid type compounds, thujone and camphor, are found at high levels and may cause potential side effects. Medicinal and food teas made from dried sage may contain as much as 30 mg/L of thujone (Walch et al. (2011) Chem Cent J. 2011 Jul 21;5:44). In contrast, the products and compositions of the present invention are derived from *S. officinalis* cultivars identified as having, amongst other properties, a total thujone content which falls below the FDA accepted safe level of around 8.75mg/day for an average human of 70kg body mass. In specific embodiments of the invention the amount of total thujone present in the composition of the invention is not more than 1 ppm (1mg/kg). In a further embodiment of the invention, in addition to the low thujone content, the amount of camphor present in the composition is less than 25 ppm, suitably not more than 10 ppm, optionally at most 7 ppm (7mg/kg). Surprisingly, the reduction in thujone content seen in these identified *S. officinalis* cultivars does not reduce the biological activity; on the contrary, a significant *increase* in potency is seen in biological assays.

Epigenetics concerns the transmission of information from a cell or multicellular organism to its descendants without that information being encoded in the nucleotide sequence of genes. Epigenetic controls are typically established via chemical modification of the DNA or chromatin structure, or via interactions between non-coding nucleic acids with chromatin. Gene expression can be moderated, in some cases, via the covalent attachment of chemical groups to polypeptides that are associated with or that can bind to DNA. By way of example, methylation, sumoylation, phosphorylation, ubiquitylation and/or acetylation of histones can lead to activation or silencing of gene expression in the region of the genome where these epigenetic modifications have occurred. Epigenetic modifications can occur at different times in the normal development and ageing of an organism, and also during transformation of normal cells into cancerous cells. Such modifications often result in the silencing or activation of certain genes. In cancer, it is well documented that the majority of tumour cells display abnormal DNA epigenetic imprints (Feinberg AP & Vogelstein B, (1983) Nature 1(5895):89-92).

By the term "modulator" it is meant a molecule (e.g. a chemical substance / entity) that effects a change in the activity of a target molecule (e.g. a gene, enzyme etc.). The change in activity is relative to the normal or baseline level of activity in the absence of the modulator, but otherwise under similar conditions, and it may represent an increase or a decrease in the normal / baseline activity.

Human ageing is associated with a series of complex multi-system conditions and diseases such as neurodegenerative disease, cognitive decline, cancer, osteoporosis, sarcopenia, in addition to cosmetic defects including skin disorders and hair loss. At the route of all age-related conditions is molecular and cellular ageing, which leads to the loss of function and eventually viability of individual cells. It is known that many aspects of cellular ageing are mediated via epigenetic mechanisms.

Separate biological ageing processes are also considered to take place in human cells: the replicative life-span of the cell (RLS) -that is how many times the cell will divide, and chronological life-span (CLS) - that is how long a non-dividing cell remains viable. Significantly, stimulated increases in the replicative and/or chronological lifespan of model organisms can not only delay, but even reverse, a host of age-related conditions and pathologies in humans and animals including cancers, neurodegenerative disease (e.g. Alzheimer's Disease and Parkinson's Disease), osteoporosis, sarcopenia, as well as cosmetic conditions of the skin. Without wishing to be bound by theory, it is proposed that in relation to the present invention the unique formulation comprises an epigenetic modulatory activity that delays or reverses epigenetic modifications that occur in the course of normal cellular ageing. The effects of this epigenetic activity are shown to have profound effects upon cell viability that results in an unexpected increase in chronological lifespan.

Many of the epigenetic pathways known to affect the biological processes associated with aging and longevity have been conserved through evolutionary history across a range of species, thus allowing meaningful, transferrable results to be obtained from model organisms and applied to human biology.

*Caenorhabditis elegans* is a small (∼1mm long), free-living, non-parasitic nematode worm. It can be cultivated in large numbers in liquid medium or on agar plates, and due to its transparent cuticle, it is possible to track cells and follow biological processes. Unlike more complex model organisms, *C. elegans* has a short life cycle (3 days from egg to reproducing adult), which considerably reduces the amount of time it takes to produce initial results of drug screens, making it a popular, cost effective choice for pharmaceutical use. The simplicity of the organism does not, however, mean that it is any less valuable as a model organism; it is estimated that 60-80% of *C. elegans* genes are homologous to those in humans.

*C. elegans* is used as a disease model for neurodegenerative disorders, muscle-associated disorders and cancer and can be manipulated for the transgenic expression of human disease genes. The validity of *C. elegans* as a model organism for research into human biology was recognised in the 2002 Nobel Prize in Physiology or Medicine. It is also a leading model for research into mechanisms of ageing. The easy manipulation of *C. elegans* genes means that mutant strains can easily be produced to optimise research efficiency. Consequently, there has been extensive research into *C. elegans* as a paradigm for Alzheimer's disease (Ewald and Li (2010), Brain Struct Funct. Mar; 214(0): 263-283)

Executive function of the brain is known to decline with age and benefit from a higher level of cardiorespiratory fitness. Anti-ageing effects induced in neuronal tissues could have direct effect not only on general fitness of the organism but also on cognitive performance. Hence, there is a clear linkage between compositions that are able to induce an increased chronological cellular lifespan and improvement in cognitive function.

Both normal aging and dementia are linked to a diminished cholinergic functioning, and it is therefore not surprising that most of the research to identify small molecule drugs which could potentially treat or alleviate the effects of severe dementia such Alzheimer's disease, have been mostly focusing on the cholinergic system. Regarding the latter, two types of receptor - muscarinic and nicotinic - respond to acetylcholine to facilitate intracellular communication, memory processing and higher cognitive functions. The process begins when acetylcholine is released to travel across the synaptic cleft where it binds to a receptor on the other side of the synapse. Once the signal is triggered acetylcholine is rapidly broken down by an enzyme, acetylcholinesterase (AchE) and made available to be recycled. Hence, conventional medical treatment for dementia involves AChE inhibitors which allow the neurotransmitter to persist in the synaptic cleft for a longer period of time.

The three most commonly prescribed AChE inhibitors are Donepezil (Aricept™), Rivastigmine (Exelon™) and Galantamine (Reminyl™), which were approved in 1996, 2000 and 2001 respectively. Donepezil is the only AChE inhibitor approved for the treatment of all stages of Alzheimer's disease (Ng YP (2015) Neurochem Int. Oct;89:260-70). *Salvia officinalis,* has also been shown to present a concentration dependant inhibition of AChE in post-mortem brain homogenates, both with essential oils and alcoholic extracts of fresh or dried leaf. Although, the AChE inhibition properties of *Salvia officinalis* are believed to be around two orders of magnitude less than that of the AChE inhibitors of Donepezil, Rivastigmine and Galantamine (Scholey et al. (2008) Psychopharmacology 198:127-139). In embodiments of the present invention botanical extracts of particular cultivars of *Salvia officinalis* with a distinct and identifiable chemical fingerprint are shown to have profound effects on chronological lifespan in *C. elegans,* even outperforming all of the approved small molecule AChE inhibitors mentioned previously. According to one embodiment of the invention, these unexpectedly significant effects on chronological lifespan correlate to cognition-enhancing properties that may be useful in treatment or remediation of those suffering from dementia related illnesses (such as Alzheimer's disease) or even in otherwise healthy subjects. Hence, the invention provides analytics as well as functional methodology, based upon an established model biological assay, for identifying the cultivars of *Salvia officinalis* with the most desirable properties and biological activity.

In use, the compositions of the invention can be administered to a human recipient as a daily controlled dose formulation. In one embodiment of the invention, the composition is administered in the form of an orally disintegrating tablet, which dissolves quickly and has a high dose capacity. The compositions of the invention are formulated to conform to regulatory standards and typically are administered orally or via other standard routes. The pharmaceutical preparations may be in the form of tablets, pills, capsules, lotions, gels, liquids, powders, suppositories, suspensions, liposomes, microparticles or other suitable formulations known in the art. Such compositions will typucally further comprise pharmaceutically acceptable excipients.

A pharmaceutically acceptable excipient or carrier refers to one or more substances that aid the administration of the active component to and absorption by a subject and can be included in the compositions of the present invention without causing a significant adverse toxicological effect on the subject. Non-limiting examples of pharmaceutically acceptable excipients include water, NaCl, normal saline solutions, lactated Ringer's, normal sucrose, normal glucose, binders, fillers, disintegrants, lubricants, coatings, sweeteners, flavors, salt solutions (such as Ringer's solution), alcohols, oils, gelatins, carbohydrates such as lactose, amylose or starch, fatty acid esters, hydroxymethycellulose, polyvinyl pyrrolidine, and colors, and the like. Such compositions can be sterilized and, if required, mixed with additional auxiliary agents such as lubricants, preservatives, stabilizers, wetting agents, emulsifiers, salts for influencing osmotic pressure, buffers, coloring, and/or aromatic substances and the like that do not deleteriously react with the active component. The skilled person will recognize that other pharmaceutical excipients are useful in the present invention.

According to one embodiment of the invention the composition may be administered at least daily - in one or more dosages - and comprise by dry weight not less than about 1 mg of the active component. Typically, the composition will comprise not less than about 10 mg of the active component, optionally not less than about 50 mg of the active component, suitably not less than about 100 mg of the active component. In an embodiment of the invention, the composition is administered at least daily - in one or more dosages - and comprises not more than about 25g of the active component, suitably not more than about 10g, and optionally not more than about 1g (i.e. 1000mg). In a specific embodiment of the invention, individual dosages of the active component may be formulated in bolus amounts of not less than about 100mg, not less than about 200mg, not less than about 400 mg, and not less than 500 mg. In a specific embodiment of the invention, a dose of 333 mg of the active component is administered to an adult subject once, twice or three times daily. In a further embodiment of the invention a dose of 167 mg of the active component is administered to an adult subject once, twice or three times daily.

Multiple dosages may be administered to an individual in order to reach the required amount to achieve a suitable daily dosage regimen. The dosage regimen, including the amount of active component administered, may vary dependent upon factors such as but not limited to: age, body mass, body mass index (BMI), metabolic profile, gender, ethnic origin, and previous medical history.

The formulation of the invention can be comprised in a liquid, emulsion or powdered solid form that can be utilised as an ingredient in other food products. For example, a staple of many current diet regimes is the protein shake that acts a meal replacement. The formulation of the invention can be conveniently incorporated into such a product so as to provide a convenient nutraceutical enhancement to the product. Other foodstuffs that can comprise the formulation of the invention suitably include cereals, soups, ready meals, mousses, yoghurts, beverages, smoothies, flours, snack foods, biscuits (cookies), cakes, breads and baked goods, confectionery, jams, spreads, ice creams, chewing gum, sauces, and salad dressings. In this way the desirable activity exhibited by the compositions of the invention can be provided at the same time as food intake.

The compositions of the invention are not limited to use in humans, veterinary compositions are also considered as a suitable means for facilitating lipid health in animals. Mitigation and prevention of age-related pathologies is of great importance in increasing the effective performance life of sporting animals such as thoroughbred racing horses and polo ponies. Just as diseases associated with ageing are increasing in prevalence as humans live longer, it is also becoming more common in domestic pets such as dogs and cats. Compositions of the invention intended for veterinary use can be administered to animals as nutraceutical formulations or alternatively the formulation can be incorporated into animal feedstuffs. It will be appreciated that dosage levels will be adapted to reflect body mass (or BMI) of the animal to which the composition is administered.

The invention is further illustrated by the following non-limiting examples.

### EXAMPLES

### Example 1

### Identification and characterisation of Salvia officinalis of unique origin

The N92 Product is produced from a highly specific Target Raw Material (TRM). This TRM is the dried clean leaf of a selected varietal of *Salvia officinalis,* grown at specific geographical locations within the United Kingdom to defined production protocols meeting Good Agricultural Production (GAP) standards and harvested within a specific seasonal window.

This TRM, as defined, can be differentiated from all other material on the basis of the characterisation of the complete chemical profile of the crude extract produced by solvent extraction - that is, differentiated from material of different species, of the same species grown elsewhere and of the same the same genetic material as the TRM grown under the same conditions as the TRM at the same locations as the TRM but harvested outside the defined seasonal harvest period for the TRM. The N92 Product is, in part, characterised as having very low content of the monoterpenoid thujone (typically no more than 1 ppm as determined by gas chromatograph analysis), considerably lower than that normally found in cultivated and wild *S. officinalis.*

Material from the N92 Unique Production Sites (UPSs) is used to define a N92 target raw material (TRM) specification as prescribed by standard operating procedures. The process in outline is as follows: NMR spectra of test extracts of TRM samples from commercial supplies of acceptable N92 raw material are subjected to a mathematical transformation to generate statistically tractable meta-data. These data are then submitted to multivariate analysis to determine the principal components of variation of the spectral data (Principle Components Analysis or PCA is a standard method of achieving this end). From this data and the mathematical models generated to describe it, a "sphere of acceptability" (in fact a multidimensional volume determined e.g. by the principle components of the test data) is generated which encloses the TRM data and which forms a basis for both visual and statistical assessment of the acceptability of new or unknown samples. This sphere of acceptability describes the, or part of the, standard specification for the N92 TRM.

Examples are given of three types of tests to which the target raw material (TRM) has been subjected, namely comparisons between the TRM and non-target species (NTS), between TRM and non-target production (NTP) samples of N92 (non-target varieties and locations) and TRM and acceptable limits to material specification or Parameters (out-of-season harvest in the example given below)

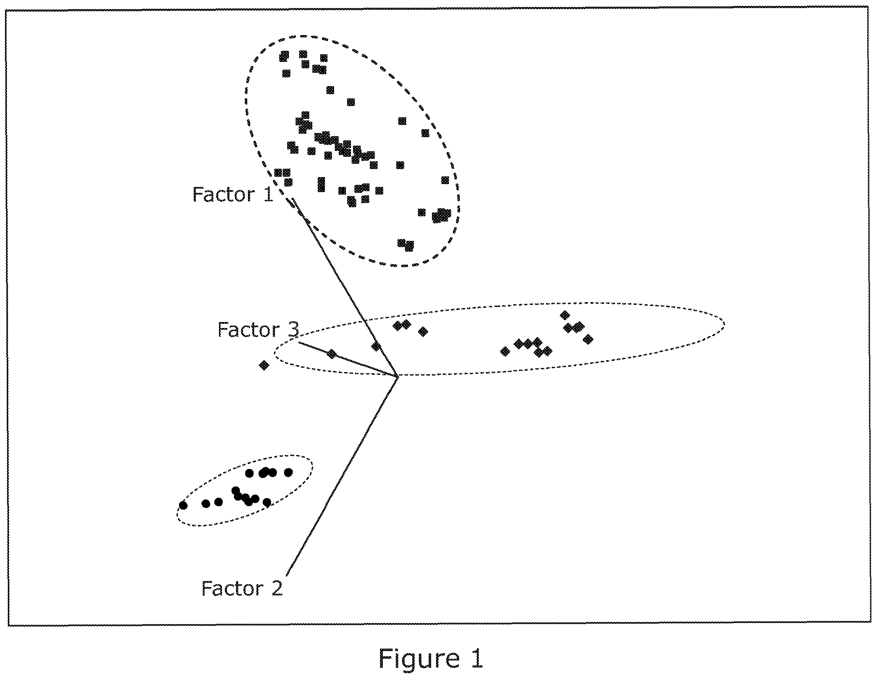

TRM data includes all data from N92 commercial harvests to date. NTS are *Salvia fructicosa* and *Salvia lavandulifolia.* The multivariate analysis technique used to produce Fig. 1 is Soft Independent Modelling of Class Analogies (SIMCA). There is clearly no overlap between the ellipsoids showing confidence limits for the TRM (circled by dashed line) and the NTS materials. The differences between the groups are examined in further detail below.

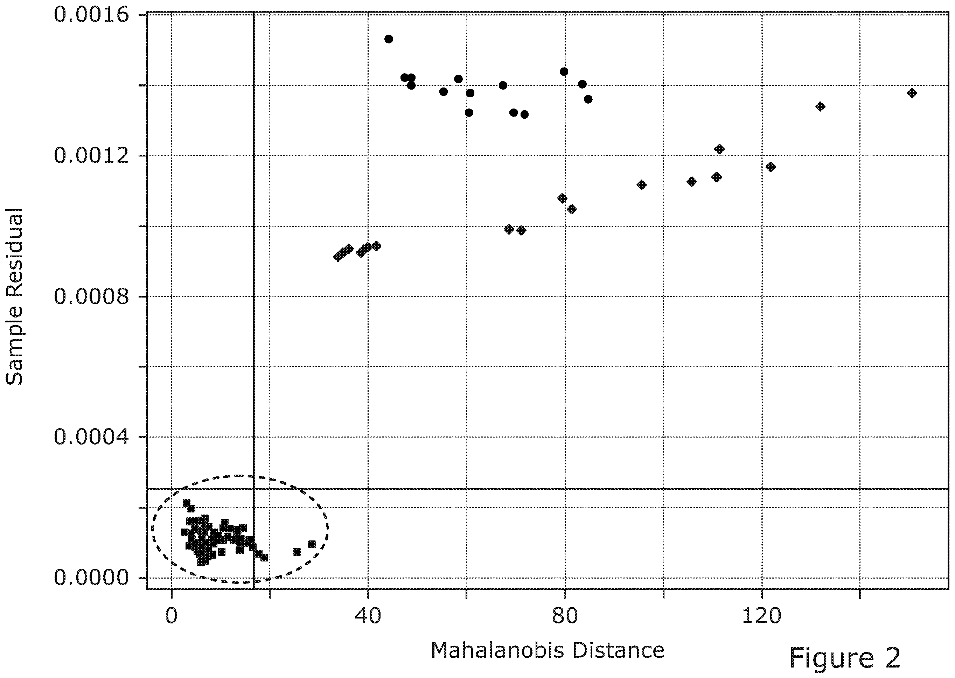

A validated Principal Components model was created to describe the TRM data and run against the NTS data; the resultant data is used to generate an outliers diagnostic chart (Fig. 2) divided into four quadrants. The X axis shows the mahalanobis distance (a measure of the distance of tested data from the modelled peaks in the NMR spectrum) and the Y axis shows the residual distance (residual variance in the NMR spectrum). Samples exceeding a critical distance (in Fig 2, this is based on a 95% confidence limit) along either axis are potential outliers; however, in a large dataset some normal samples may fall outside one or the other threshold. Samples falling significantly outside both thresholds clearly differ from the modelled population. This example (Fig 2) shows that the NTS samples are distinctly materially different from those of the TRM (circled by dashed line).

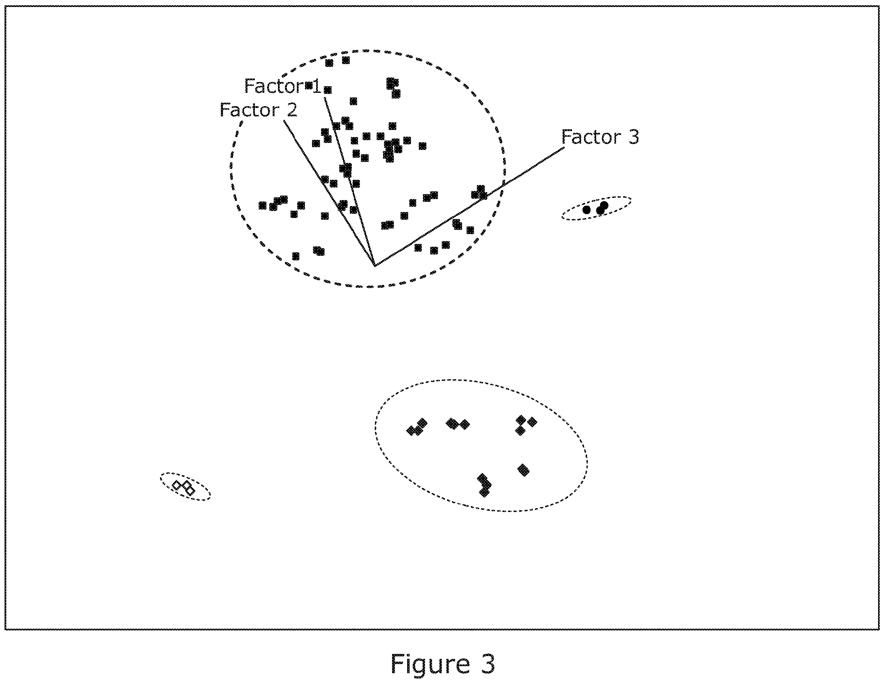

In Fig.3, TRM (circled by dashed line) includes all data from commercial harvests to date compared to the NTP's which include varieties from the UK (bottom right), Croatia (bottom left) and Italy (top right). There is clearly no overlap between the ellipsoids indicating confidence limits for the TRM and the NTP materials.

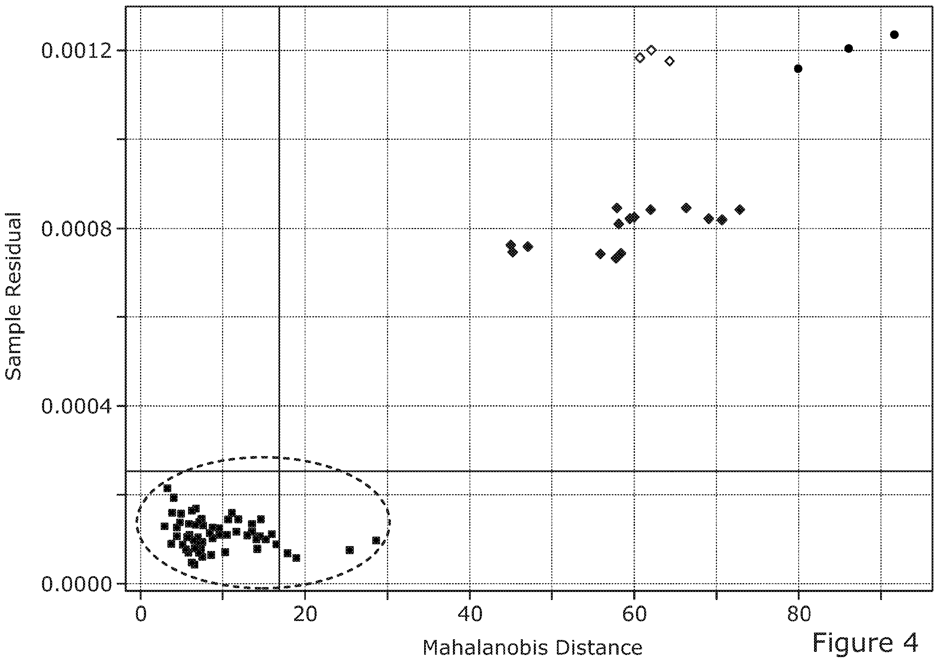

A validated Principal Components model was created to describe the TRM data and run against the NTP data; the resultant data is used to generate an outliers diagnostic chart (Fig 4): divided into four quadrants. Samples falling outside both the x- and y- critical thresholds are significantly different from the modelled population. The graph shows that the NTP samples are distinctly materially different from those of the TRM (circled by dished line).

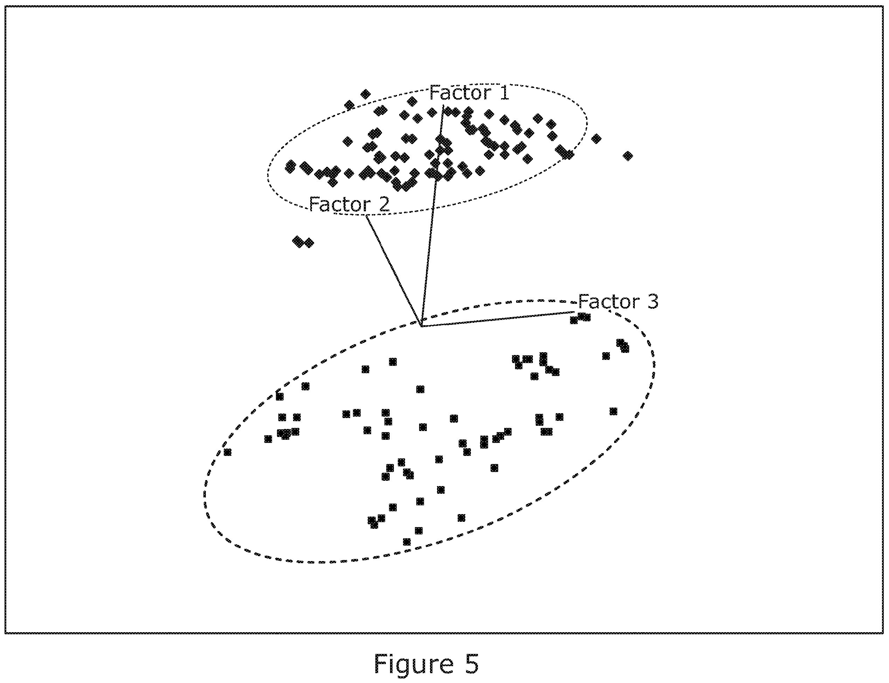

In Fig.5, TRM (bottom, circled by dashed line) includes all data from commercial harvests to date. Limits-to-specification material (Red) was harvested outside of the prescribed season, and differs phytochemically from the desired TRM, as is demonstrated above (the confidence limits for the two groups do not overlap).

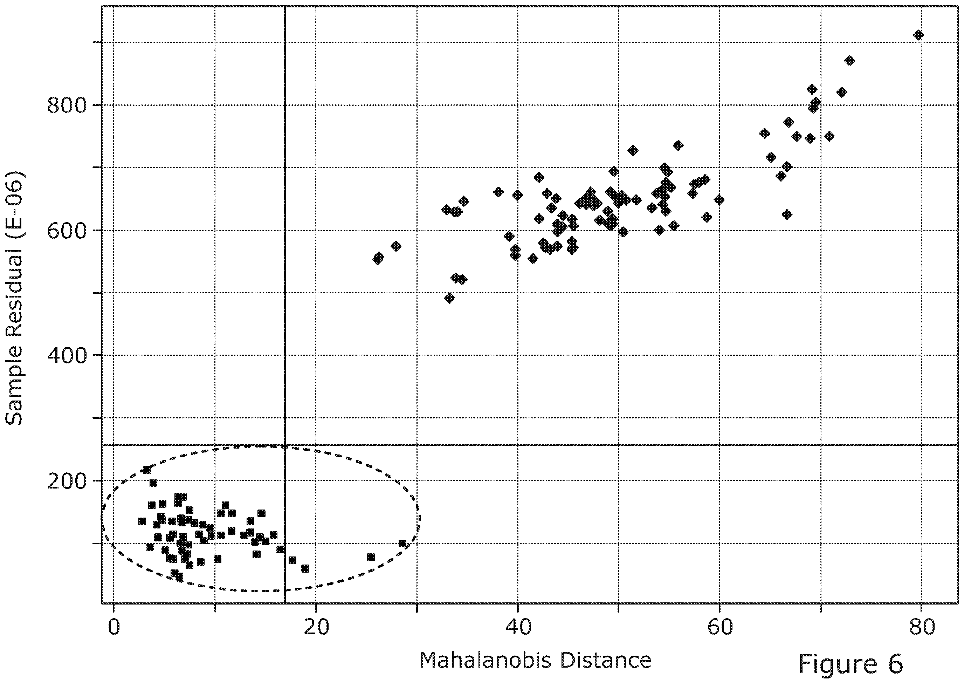

Fig 6 shows a validated Principal Components model was created to describe the TRM data and run against the NTP data; the resultant data is used to generate an outliers diagnostic chart divided into four quadrants. Samples falling outside both the x- and y- critical thresholds are significantly different from the modelled population. Our example shows that the out of season samples are distinctly materially different from those of the TRM.

The methodology set out, therefore, provides a reproducible chemical fingerprint that enables identification of *S. officinalis* TRM independent of variety, location of cultivation or season of harvest.

### Example 2

The leaf is mechanically harvested and dried in a hot air drier (continuous process drier; indirect fired heating). Leaf material came from the 2011 harvest cultivated at a specified location in the United Kingdom.

### Salvia officinalis - TRM ethanolic/water extraction protocol

- Grind up 10g of TRM using Mortar and Pestle
- Weigh out 0.1g into an 6x Eppendorf tube
- Add 600 µl of 70% EtOH to each tube
- Incubate shaking at 39 degrees Celsius for 3 hours
- Combine tubes and filter sterilise using a 0.2 µm filter and 5 ml syringe
- Pipette 1.5 ml into a 1.5 ml tube and centrifuge for 1 min at 13k rpm and transfer supernatant to a fresh tube.
- Evaporate solvent by placing in speed vac (40 degrees Celsius) for 3-5 hours. The resulting dried powdered ethanolic TRM extract is in the form of a fine green-brown powder.
- Resuspend dried TRM extract in DMSO at a concentration of 10 mg/ml.

### Example 3

N92 Product comprises the resuspended dried TRM extract produced in Example 2 and was used as the sage source in the present Examples. All of the ingredients used in the examples were formulated as 10 mg/ml DMSO stocks and used accordingly. N92 showed significant increases in median lifespan over untreated controls in both yeast and nematode worm screens. Screening was carried out in accordance with the protocols described in

### Yeast and Worm Single Compound Screening

A- All described ingredients were tested alone against appropriate controls to determine efficacy in the Yeast Screen.
B- The most attractive ingredients from Stage A were tested again in the worm screen to ensure no yeast specific effects were being observed.

### (A) Single Composition Yeast Screening

Screening was carried out substantially in accordance with the protocols set out in WO-2011/004197-A1. Yeast were grown at 30°C in rich medium (YPD), 1 % bactopeptone, 1% Difco yeast extract (BD and Co.), 2% glucose to a density of 0.4 x 106 cells/ml and treated with N92 product or mock treated for up to three hours. Lifespan was determined as described in Murakami, C. and Kaeberlein, M. (J. Vis. Exp., 27 (2009)). Briefly, chronological lifespan of yeast refers to the profile of viability of an ageing yeast culture over time. A yeast culture is grown in liquid media until the glucose carbon source is exhausted and the cells stop dividing. At this point the proportion of cells which are alive and therefore able to grow and divide upon supply of fresh nutrients is measured by observing the outgrowth characteristics of a fresh inoculate of the aging culture using a Bioscreen C machine (Oy Growth Curves Ab Ltd, Helsinki, Finland). Viabilities at various time points are compared to determine the chronological lifespan of the culture.

N92 (Sage) was run twice in the yeast screen, once at a 1/10 dilution. In both experiments the results show a significant extension in median lifespan over the untreated control culture.

**Table 1 N92 (S. officinalis) gave up to an 18% extension in median lifespan in yeast.**

| | **Conditions** | **Increase in Median Lifespan** |
|---|---|---|
| **N92 Sage** | **N92** | 17.95% +/- 0.14 |
| | **N92 1/10** | 7.69% +/- 0.08 |

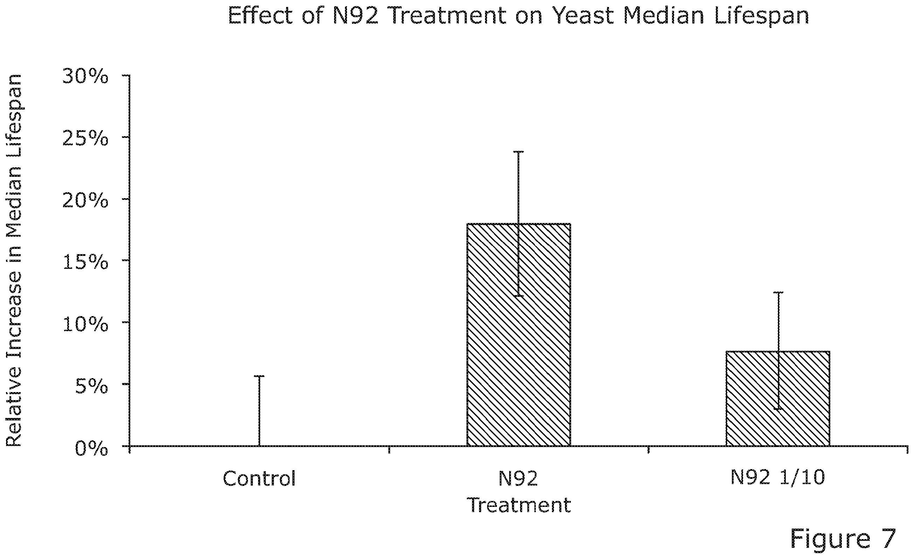

The results are also shown in Fig. 7.

### (B) Single Composition Worm Screening

N92 Worm Screen - the screen was carried out in the nematode, *C. elegans* substantially in accordance with the protocols set out in WO-2014/111732-A1 (Chronos Therapeutics Ltd). Nematode worms were grown on an agar plate using standard procedures in order to obtain sufficient eggs at the start of the experiment. Plates are kept in the incubator at 20 degrees Celsius.

The N92 test compositions at standard, 1/10 and 1/100 dilution were prepared and applied to pre-poured 12-well agar plates. A minimum of 4 replicates per condition including control were used. Once the test compositions had dried, nematode feed bacteria were applied to the plates (*E*. *coli* OP50 strain).

Worms and eggs previously placed in the incubator were collected. Typically this involves rinsing the plates with M9 buffer and then performing 3 cycles of centrifuge while bleaching the worms (so that worms are killed and only the eggs survive). Once completed the egg concentration is calculated and diluted as necessary to dispense 200 eggs per well. As approx. 75% of eggs survive this results in around 150 worms per well which is a stable number considering the volume of the well and food availability. The plates were incubated for 48 hours at 20 degrees Celsius to enable hatching.

The incubated worms are sterilized with FUDR (2'-Deoxy-5-flurouridine), a chemotherapeutic drug which is used in *C. elegans* studies to prevent the formation of second generation progeny which would otherwise complicate ageing experiments. FUDR prevents DNA synthesis and therefore eggs do not hatch and young worms do not develop.

The cultures were then regularly imaged by bright field microscopy on days 1, +3; +4; +7; +9; +11; +14; +16;+18. The worms were fed with OP50 as necessary, typically dispensing *E.coli* each 2-3 days to prevent population decline as a result of starvation.

Once imaging was finished the data was analysed with an in-house MatLab software package, and an Excel spreadsheet was produced as output and analysed by conventional statistical software to plot the survival curves for each of the cultures.

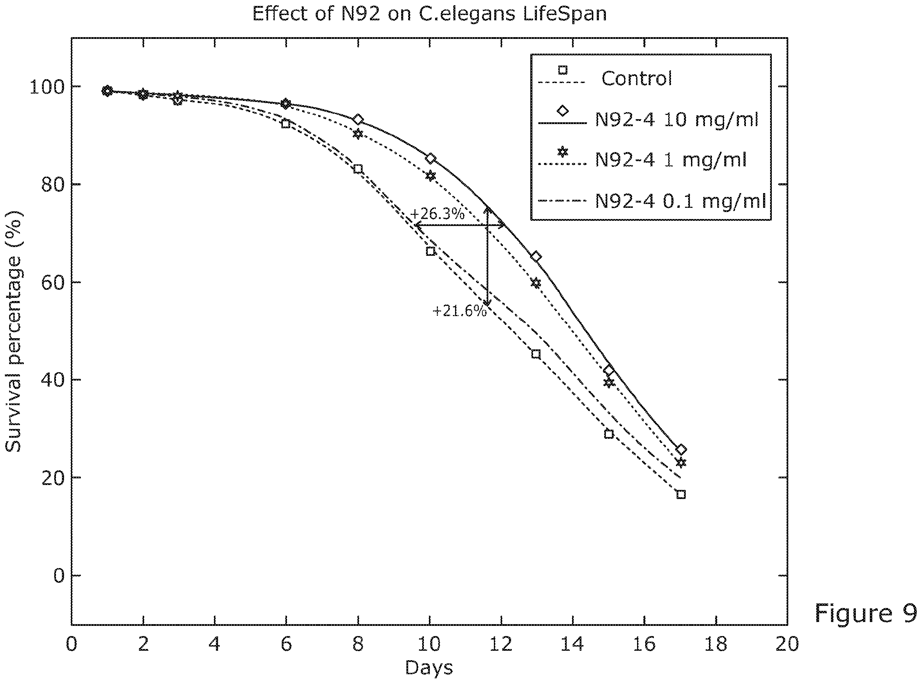

N92 product resulted in a significant increase in lifespan of worms and significant extension on mean life span by +11.8%. in comparison with the untreated control. This effect was observed at 10 mg/ml dosage and was dose dependent when tested within the range of 0.1-10 mg/ml as is shown in Fig. 9. Further analysis of these results and comparison to other compounds is shown in Example 4 (below).

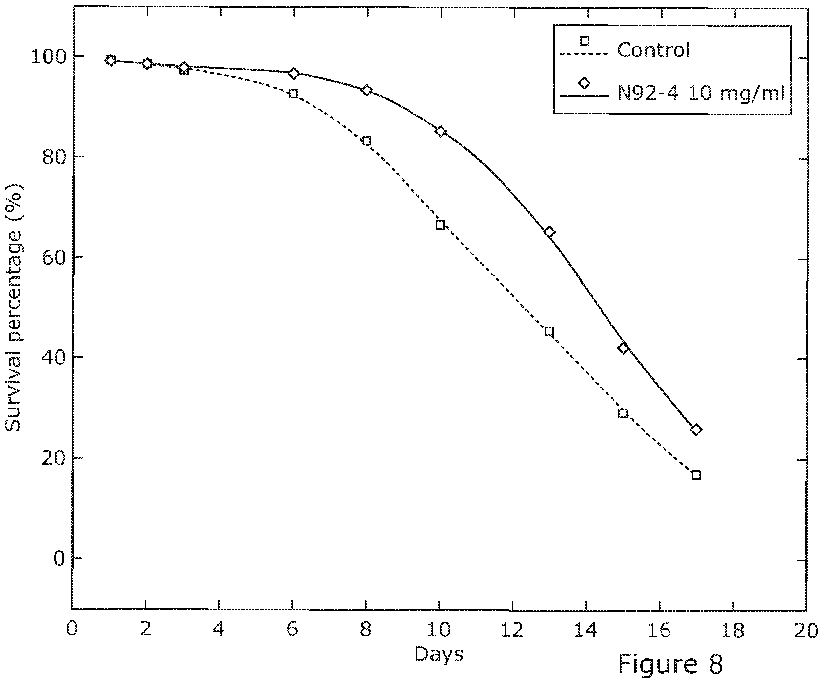

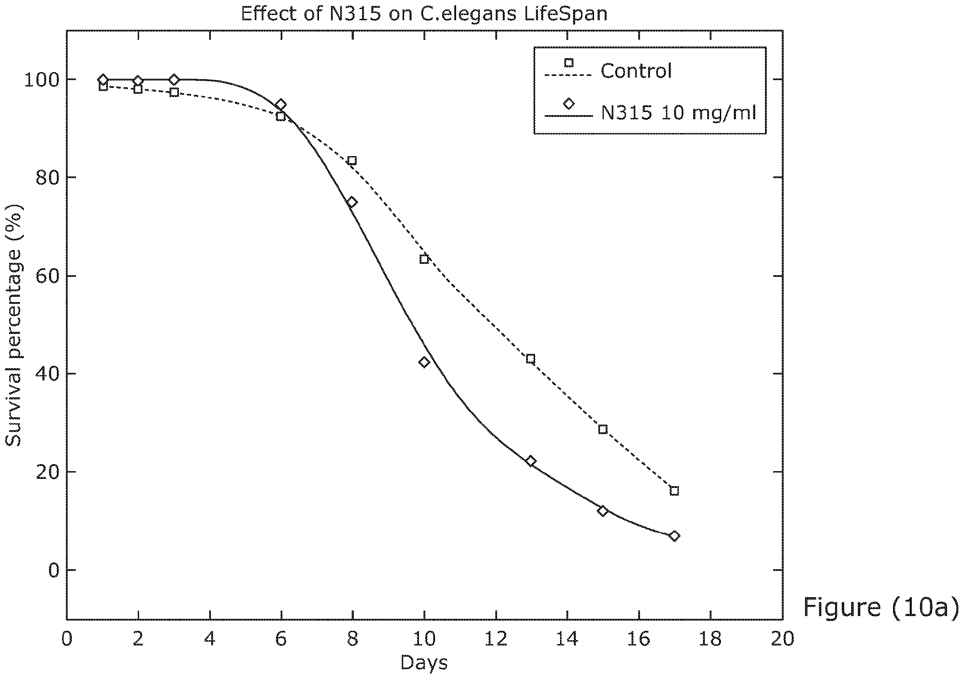

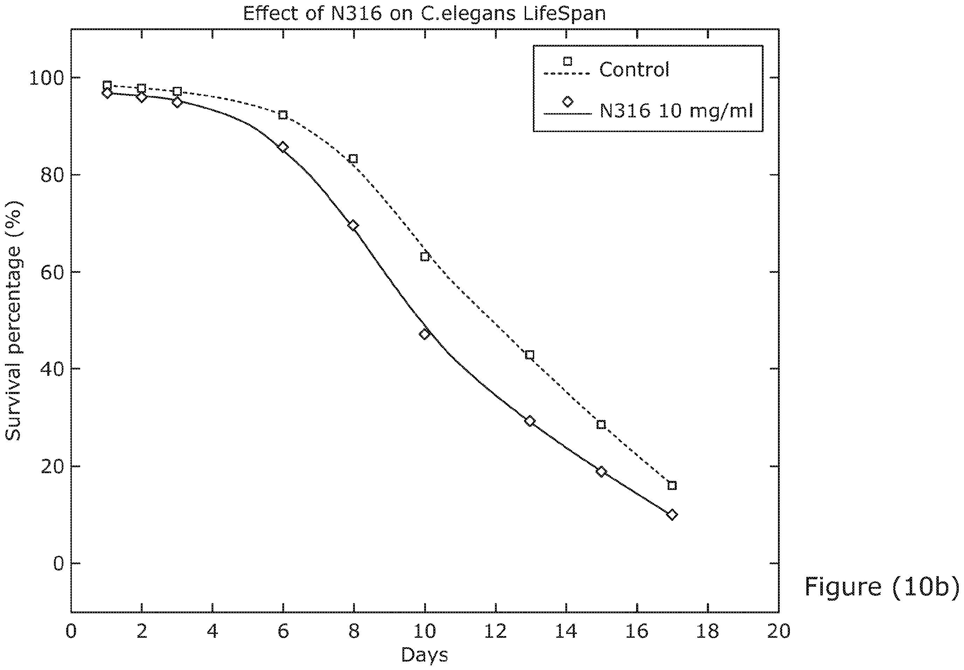

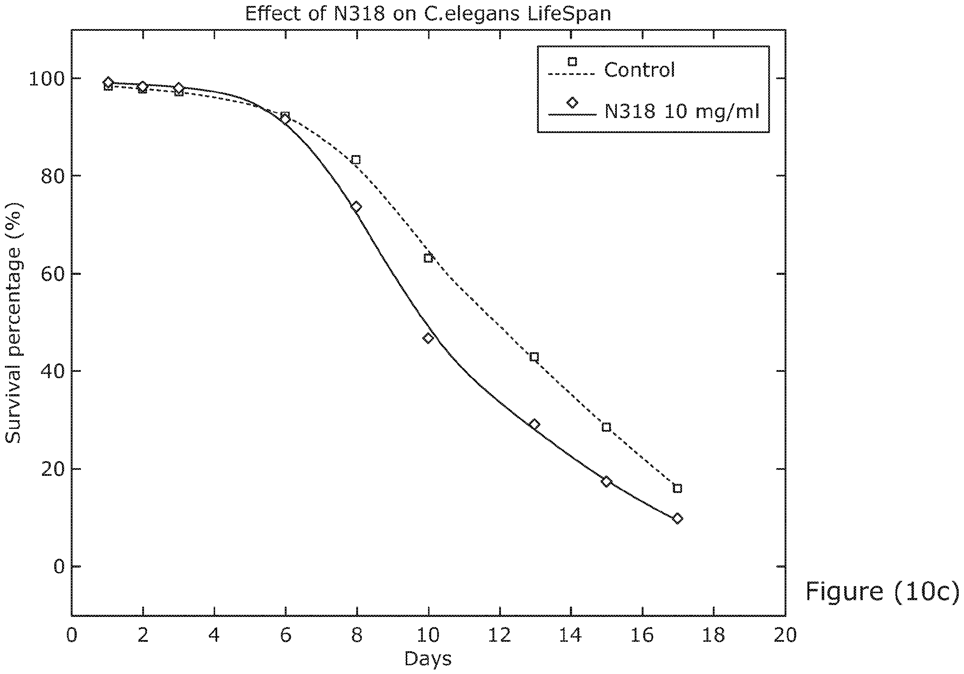

When compared with activities of alternative sages, i.e. red sage (N315), an English sage (N316) and a sage supplied from Spain (N318), it was clear that by mean life span extension, maximum life span extension and maximum extension of survival N92 was the most active. The results of the comparisons are shown in Table 2 where it can be seen that composition from N92 was the only composition that actually resulted in an observed mean life span extension, maximum life span extension and maximum extension of survival in the assay. The results of this experiment are further shown in Figures 8 and 10 (a-c).

The results of the yeast and worm biological screens clearly demonstrate that the N92 composition exerts profound effects upon chronological ageing leading to an increase in lifespan in both the model organisms tested. The robustness of these results between divergent organisms suggests that similar effects would be observed in mammalian cells. In addition, it is also important to note that the beneficial effects are not ubiquitous to compositions obtained from all sages (*S*. *officinalis*) and are unique to varieties, such as N92, which typically exhibit unusually low thujone content.

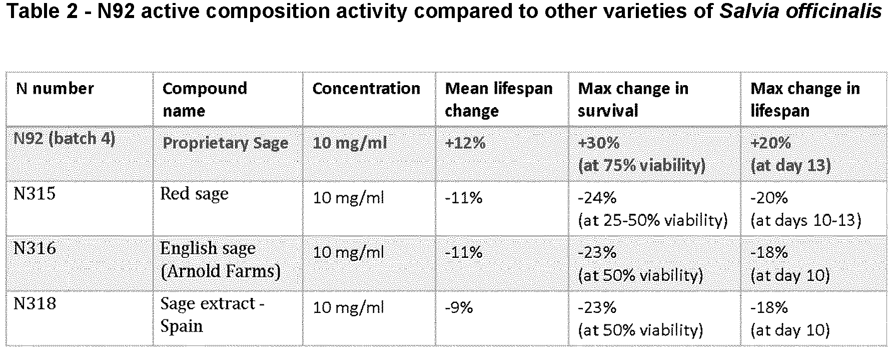

### Example 4

### Screening in C. elegans chronological lifespan assay of S. Officinalis extracts in comparison with small molecule AChE inhibitors

The three most commonly prescribed AChE inhibitors in common use for treatment of Alzheimer's disease are Donepezil (Aricept™), Rivastigmine (Exelon™) and Galantamine (Reminyl™). Botanical extracts of *S. Officinalis* from two unique sources were prepared as described previously in Example 2. The details of the compositions tested are set out in Table 3 below.

**Table 3**

| **Compound ref.** | **Compound Name** | **Compound Type** | **Supplier** | **Compound Description** | **Purity** |
|---|---|---|---|---|---|
| N350 | Donepezil | Small molecule drug | Sigma-Aldrich | Powder | ≥98% HPLC |
| N355 | Rivastigmine | Small molecule drug | Sigma-Aldrich | Powder | ≥98% HPLC |
| N354 | Galantamine | Small molecule drug | Sigma-Aldrich | Powder | ≥94% TLC |
| N314 | *Salvia officinalis* - UK Sage Source A | Botanical extract | N/A | Extract | N/A |
| N92-4 | *Salvia officinalis* - UK Sage Source B | Botanical extract | N/A | Extract | N/A |

The samples were prepared as a DMSO-based 10mg/ml stock, aliquoted when necessary and a serial dilution of the stock solution was performed for N92-4. The stock solutions were stored at - 20°C.

The assay was carried out using the standard *C. elegans* strain with a pharyngeal GFP tag and a *bus-5* mutation. The GFP tag enables images of the worms to be captured using fluorescence microscopy. The *bus-5* mutation results in the loss of 'normal' cuticle antigens which causes the cuticle of the worm to become permeable to the treatments, allowing direct uptake of treatment ingredients avoiding the protective mechanisms of the gut channel. The worms were raised in the presence of a treatment or control on 12 well NGM agar plates, and were maintained at 20°C with sufficient food (*Escherichia coli* strain OP-50).

A proprietary data analysis software package was used to analyse the effects of different active ingredients on aspects of worm ageing. The package was developed using a MATLAB image analysis algorithm which was adapted to recognise and count worms based on their fluorescence intensity above background fluorescence. A living worm is distinguished from a dead worm based on the degree of movement between two consecutive images, which enables the number of living and cumulative number of dead worms to be tracked throughout the experiment. A similar A selection of images was manually checked to ensure the software had correctly identified the number of worms. Using the online software OASIS (Yang, Nam, Seo, Han, & Choi, 2011), the MATLAB output is processed, generating Kaplan-Meier survival curves and associated statistics.

The statistical analysis output of OASIS provides Log-Rank and Fisher's Exact Test results. The Log-Rank result is a non-parametric test which compares two survival functions for the overall lifespan assay, providing a reliable p-value summarising the whole experiment. The Fisher's Exact Test calculates the significance of survival function comparisons at multiple specific time points throughout the experiment, rather than for the overall lifespan

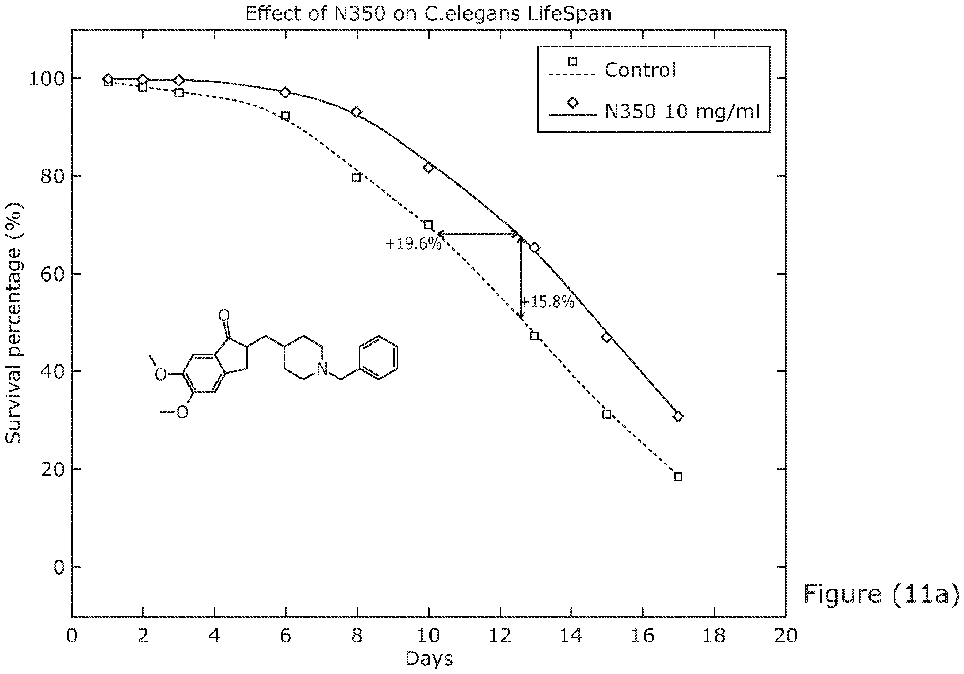

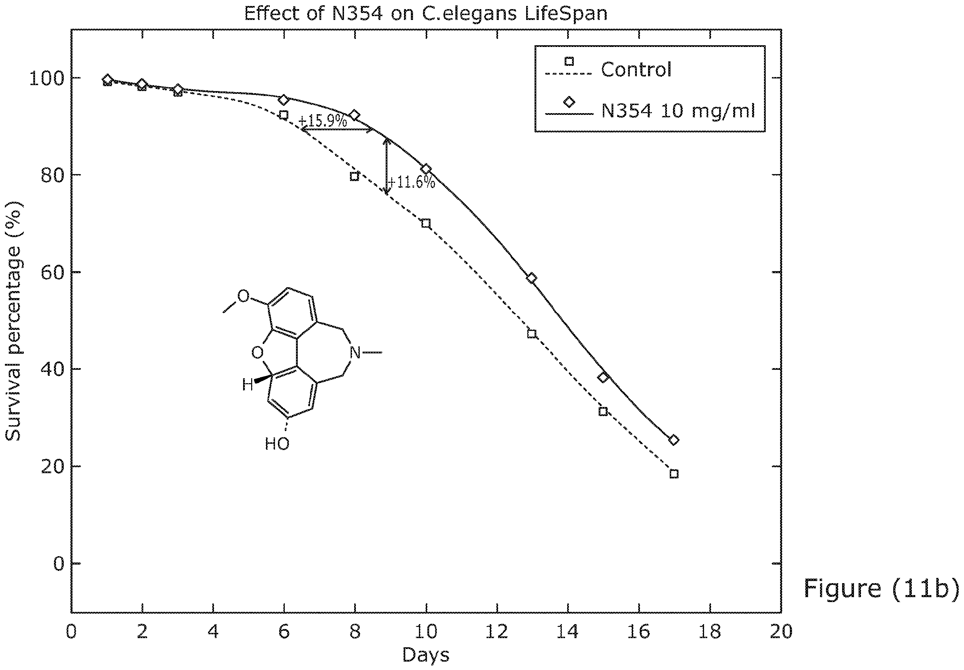

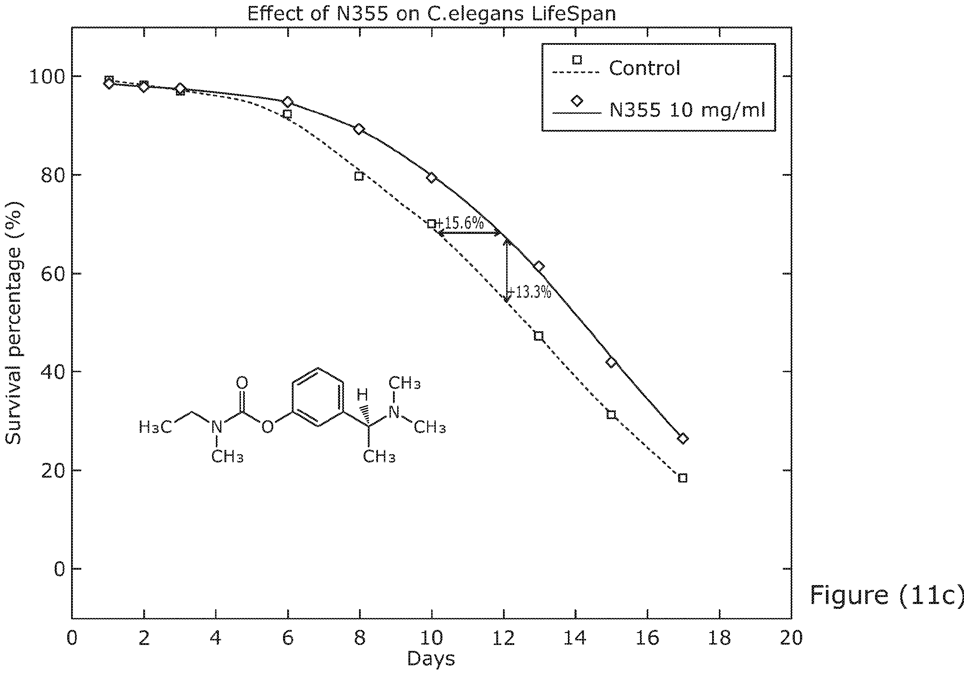

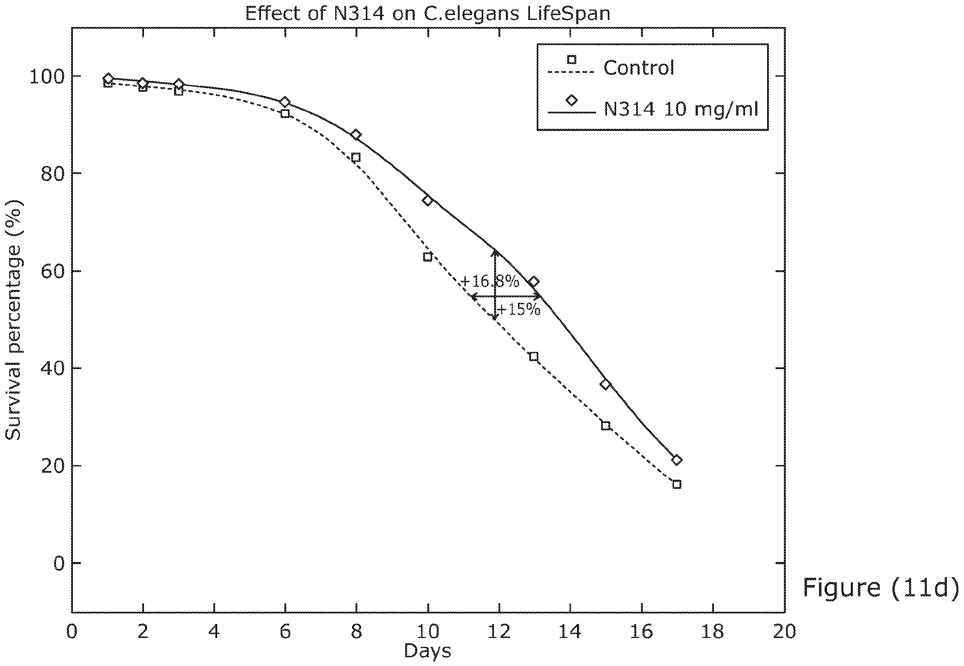

The results of the assay for each of the tested compounds are shown in Figure 11.

**Donepezil (N350)** performed with a statistically significant improvement in mean life span of +11.3%, a maximum improvement in lifespan of+19.6%, and a maximum improvement in survival up to +15.8% at day 13 (as seen in Fig. 11(a)). The results of the Log-Rank Test and Fisher's Exact Test showed significance overall and for all individual time points.

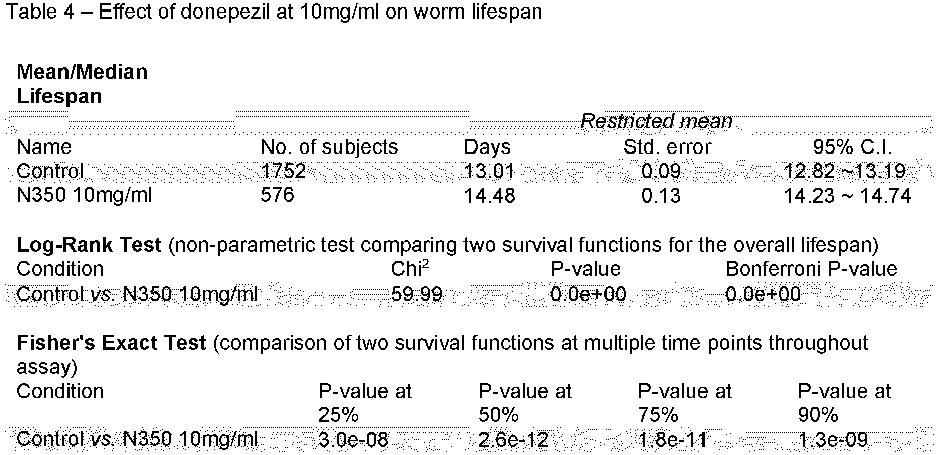

**Rivastigmine (N355)** performed with a statistically significant improvement in mean life span of +7.9%, a maximum improvement in lifespan of **+15.6%**, and a maximum improvement in survival up to +13.3% at day 12 (as seen on Fig 11(b)). The results of the Log-Rank Test and Fisher's Exact Test showed significance overall and for all individual time points.

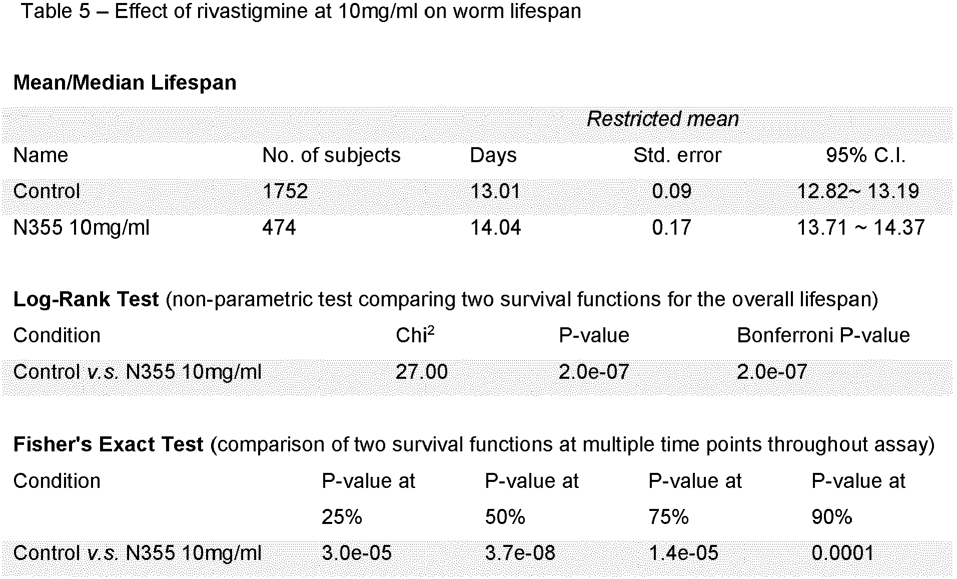

**Galantamine (N354)** performed with a statistically significant improvement in mean life span of +8%, a maximum improvement in lifespan of +15.9%, and a maximum improvement in survival up to +11.6% at day 9 (see Fig. 11(c)). The results of the Log-Rank Test and Fisher's Exact Test showed significance overall and for all individual time points.

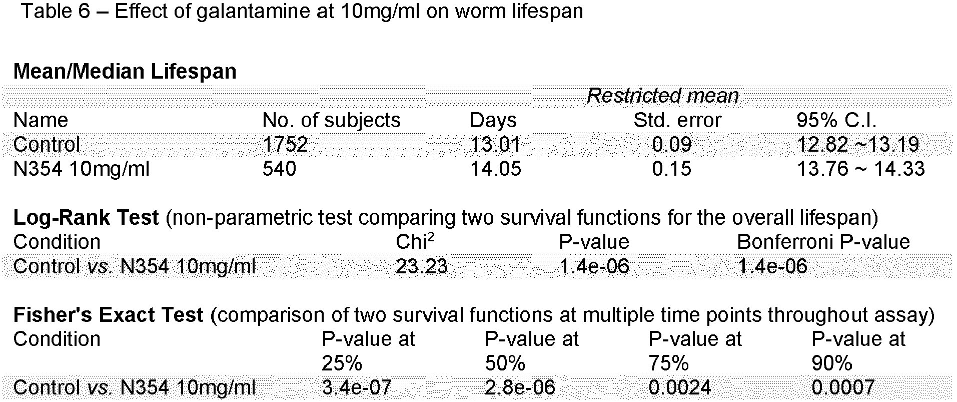

***Salvia officinalis*** - UK sage source A (N314) performed with a statistically significant improvement in mean life span of +8%, a maximum improvement in lifespan of +16.8%, and a maximum improvement in survival up to +15% at day 12 (as seen in Fig. 11(d)). The results of the Log-Rank Test and Fisher's Exact Test showed significance overall and for all individual time points.

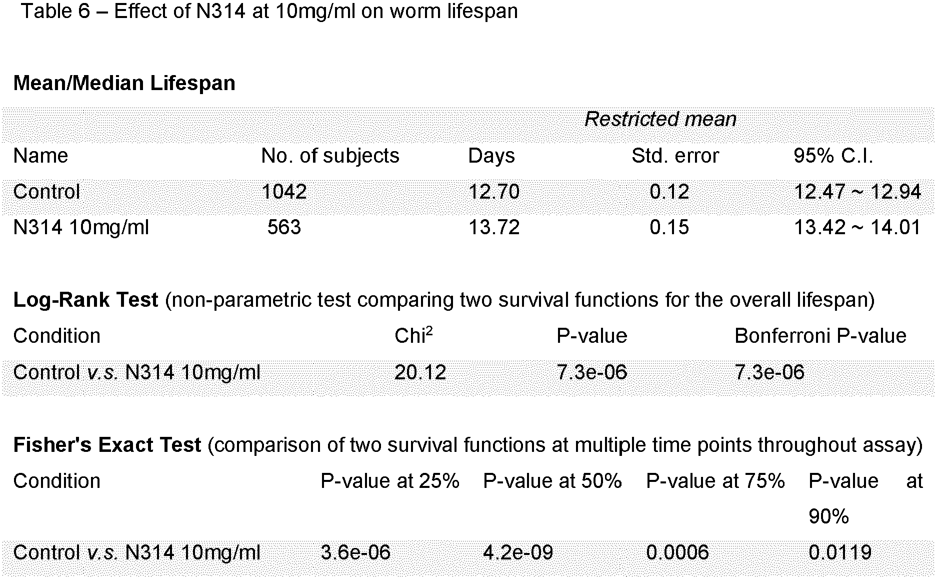

***Salvia officinalis*** - UK sage source B (N92-4), has been assessed at three different concentrations in Example 3 (see above) and showed a clear dose-dependent effect, with a statistically significant improvement in mean life span of +11.8%, a maximum improvement in lifespan of +26.3% and a maximum improvement in survival up to +21.6% at day 12 for the 10mg/ml concentration (see Fig. 9). Further analysis of the results of the Log-Rank Test and Fisher's Exact Test showed significance overall and for all individual time points at 1mg/ml and 10mg/ml.

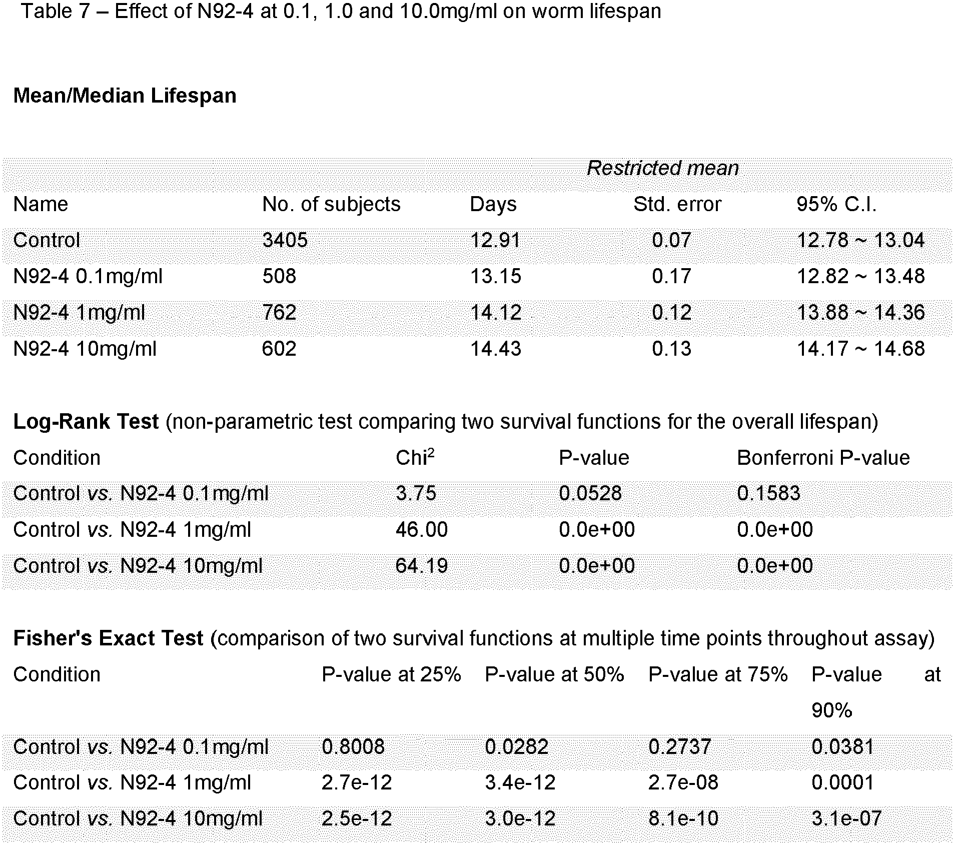

### Conclusions

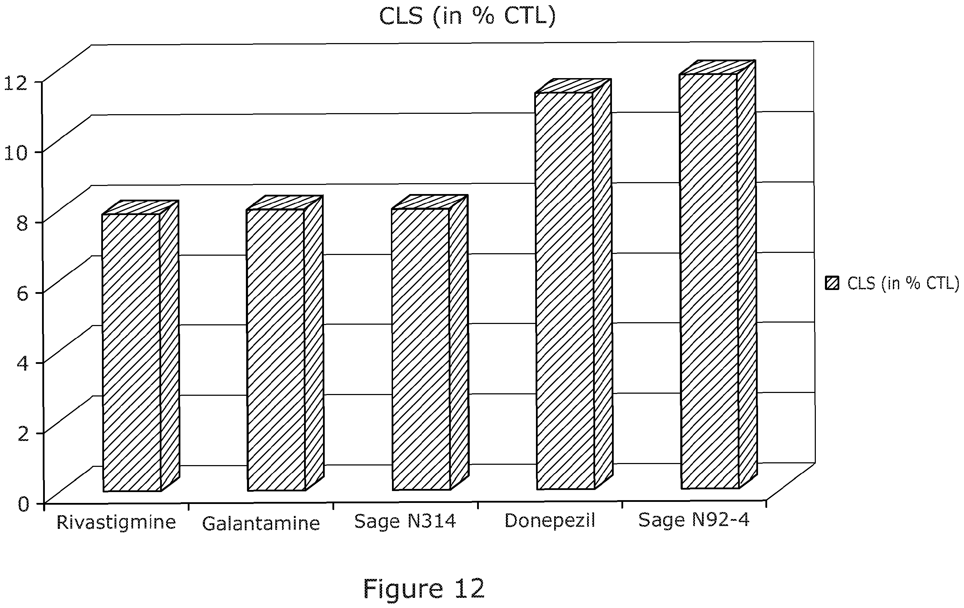

The small molecule AChE inhibitor compounds increased the chronological life span (CLS) of C. *elegans* significantly within a tight range of 8%-12%. All these drugs present an AChE inhibition activity, which tends to demonstrate that increasing the cholinergic activity in *C*. *Elegans* is associated with an extension of the CLS. *S*. *officinalis* extracts tested in comparison showed increases in CLS that were comparative to the small molecules tested. However, N92-4 surprisingly demonstrated an activity that exceeded even that of the best performing small molecule, donepezil (see Fig. 12).

Botanical extracts, while displaying a much higher variability in their properties depending on the combination of a number of factors, such as variety and cultivar, origin, production protocols and harvest times, number of years of cultivation, developmental stage of the specific harvested leaf, use of fresh or dried material, nature of the drying and extraction process and finally the duration of the storage of the plant product, may have therefore an impressive potential as therapeutic/ enhancing ingredients which could perform as well as or better than classical therapeutic drugs. By identifying a specific chemical fingerprint for a particular cultivar of *S. officinalis* that possesses advantageous biological properties the present invention provides a way of avoiding the inherent variability in plant source material that has, to date, dissuaded clinicians and nutritionists from relying upon herbal extracts for their beneficial therapeutic properties. In addition, the present *C. elegans* CLS screening approach provides a robust and repeatable assay for identification and quality control of other specific cultivars of *S. officinalis* that have equivalent or even better biological activity compared to standard crops.

Although particular embodiments of the invention have been disclosed herein in detail, this has been done by way of example and for the purposes of illustration only. The aforementioned embodiments are not intended to be limiting with respect to the scope of the appended claims, which follow. It is contemplated by the inventors that various substitutions, alterations, and modifications may be made to the invention without departing from the scope of the invention as defined by the claims.

## Claims

1. A composition comprising a nutritionally or pharmaceutically effective dosage of an extract of *Salvia officinalis* and an acceptable carrier for use in the treatment of age-related pathologies in a human or animal subject, wherein the age-related pathologies are selected from one or more of the group consisting of: cancer; neurodegenerative disease, including Alzheimer's Disease and Parkinson's Disease; cognitive decline; osteoporosis; and sarcopenia; and wherein the extract is obtained from the leaf of a variety of *Salvia officinalis* and has a thujone content of no more than around 1ppm.

2. The composition for use according to claim 1, wherein the thujone is less than around 1 ppm.

3. The composition for use according to claim 1 or 2, wherein the amount of camphor present in the composition is less than 25ppm, suitably not more than 10 ppm, optionally at most 7 ppm.

4. The composition for use according to any one of claims 1 to 3, wherein the extract is obtained from the dried leaf of the *Salvia officinalis.*

5. The composition for use according to any one of claims 1 to 4, wherein the cognitive decline comprises age-related memory impairment.

6. The composition for use according to any one of claims 1 to 5, wherein the subject is a human.

7. The composition for use according to any one of claims 1 to 5, wherein the subject is a non-human animal.

8. The composition for use according to any one of claims 1 to 7, wherein the composition is intended for oral administration.

9. The composition for use according to claim 8, wherein the composition is administered in the form of a capsule or tablet.

10. The composition for use according to any one of claims 1 to 9, wherein the composition comprises at least 1%, optionally at least 3%, by weight of the extract of *Salvia officinalis.*

11. The composition for use according to any one of claims 1 to 10, wherein the composition comprises no more than 85% by weight of the extract of *Salvia officinalis.*

12. The composition for use according to any one of claims 1 to 11, wherein the composition is administered to the subject daily, and optionally more than once daily.

13. The composition for use according to any one of claims 1 to 12, wherein the composition is administered in an amount of not less than about 1 mg of the active component, typically not less than about 10 mg of the active component, optionally not less than about 50 mg of the active component, suitably not less than about 100 mg of the active component.

14. The composition for use according to any one of claims 1 to 13, wherein the composition is administered in an amount of not more than about 25g of the active component, suitably not more than about 10g, and optionally not more than about 1g.

15. The composition for use according to any one of claims 1 to 14, wherein the composition is capable of eliciting an increase in mean lifespan in *C. elegans* worms of at least 10%, suitably at least 12%, optionally at least 20% when compared to worms that have not been exposed to the composition.

## Patentansprüche

1. Zusammensetzung, umfassend eine ernährungsphysiologisch oder eine pharmazeutisch wirksame Dosis eines Extrakts von *Salvia officinalis* und eine unbedenkliche Trägersubstanz zur Verwendung bei der Behandlung altersbedingter Pathologien bei einem menschlichen oder einem tierischen Subjekt, wobei die altersbedingten Pathologien aus einer oder mehreren der Gruppe ausgewählt sind, die aus Folgenden besteht: Krebs; neurodegenerativer Erkrankung, einschließlich Alzheimer-Krankheit und Parkinson-Krankheit; kognitivem Abbau; Osteoporose; und Sarkopenie; und wobei der Extrakt aus dem Blatt einer Sorte von *Salvia officinalis* gewonnen wird und einen Thujongehalt von nicht mehr als etwa 1 ppm aufweist.

2. Zusammensetzung zur Verwendung nach Anspruch 1, wobei das Thujon wenigerals etwa 1 ppm beträgt.

3. Zusammensetzung zur Verwendung nach Anspruch 1 oder2, wobei die Menge an Kampfer, die in der Zusammensetzung vorhanden ist, wenigerals 25 ppm, geeigneterweise nicht mehr als 10 ppm, optional höchstens 7 ppm, beträgt.

4. Zusammensetzung zur Verwendung nach einem der Ansprüche 1 bis 3, wobei der Extrakt aus dem getrockneten Blatt von *Salvia officinalis* gewonnen wird.

5. Zusammensetzung zur Verwendung nach einem der Ansprüche 1 bis 4, wobei der kognitive Abbau altersbedingte Gedächtnisbeeinträchtigung umfasst.

6. Zusammensetzung zur Verwendung nach einem der Ansprüche 1 bis 5, wobei das Subjekt ein Mensch ist.

7. Zusammensetzung zur Verwendung nach einem der Ansprüche 1 bis 5, wobei das Subjekt ein nicht menschliches Tier ist.

8. Zusammensetzung zur Verwendung nach einem der Ansprüche 1 bis 7, wobei die Zusammensetzung zur oralen Verabreichung bestimmt ist.

9. Zusammensetzung zur Verwendung nach Anspruch 8, wobei die Zusammensetzung in Form einer Kapsel oder einer Tablette verabreichtwird.

10. Zusammensetzung zur Verwendung nach einem der Ansprüche 1 bis 9, wobei die Zusammensetzung wenigstens 1 %, optional wenigstens 3 %, Gew.-% des Extrakts von *Salvia officinalis* umfasst.

11. Zusammensetzung zur Verwendung nach einem der Ansprüche 1 bis 10, wobei die Zusammensetzung nicht mehr als 85 Gew.-% des Extrakts von *Salvia officinalis* umfasst.

12. Zusammensetzung zur Verwendung nach einem der Ansprüche 1 bis 11, wobei die Zusammensetzung dem Subjekt täglich, und optional mehr als einmal täglich, verabreicht wird.

13. Zusammensetzung zur Verwendung nach einem der Ansprüche 1 bis 12, wobei die Zusammensetzung in einer Menge von nicht weniger als etwa 1 mg der aktiven Komponente, typischerweise nicht weniger als etwa 10 mg der aktiven Komponente, optional nicht weniger als etwa 50 mg der aktiven Komponente, geeigneterweise nicht weniger als etwa 100 mg der aktiven Komponente, verabreicht wird.

14. Zusammensetzung zur Verwendung nach einem der Ansprüche 1 bis 13, wobei die Zusammensetzung in einer Menge von nicht mehr als etwa 25 g der aktiven Komponente, geeigneterweise nicht mehr als etwa 10 g und optional nicht mehr als etwa 1 g, verabreicht wird.

15. Zusammensetzung zur Verwendung nach einem der Ansprüche 1 bis 14, wobei die Zusammensetzung in der Lage ist, eine Erhöhung einer mittleren Lebensdauervon *C.-elegans-* Würmern von wenigstens 10 %, geeigneterweise wenigstens 12 %, optional wenigstens 20 %, im Vergleich zu Würmern auszulösen, die der Zusammensetzung nicht ausgesetzt waren.

## Revendications

1. Composition comprenant une dose nutritionnellement ou pharmaceutiquement efficace d'un extrait de *Salvia officinalis* et d'un support acceptable pour une utilisation dans le traitement de pathologies liées à l'âge chez un sujet humain ou animal, dans laquelle les pathologies liées à l'âge sont choisies parmi un ou plusieurs du groupe composé de : cancer; maladies neurodégénératives, y compris la maladie d'Alzheimeret la maladie de Parkinson ; déclin cognitif ; ostéoporose ; et sarcopénie ; et dans laquelle l'extrait est obtenu à partir de la feuille d'une variété de *Salvia officinalis* et a une teneur en thujone non supérieure à environ 1 ppm.

2. Composition à utiliser selon la revendication 1, dans laquelle la thujone est inférieure à environ 1 ppm.

3. Composition à utiliser selon la revendication 1 ou 2, dans laquelle la quantité de camphre présente dans la composition est inférieure à 25 ppm, convenablement pas plus de 10 ppm, éventuellement au plus 7 ppm.

4. Composition à utiliser selon l'une quelconque des revendications 1 à 3, dans laquelle l'extrait est obtenu à partir de la feuille séchée de *Salvia officinalis.*

5. Composition à utiliser selon l'une quelconque des revendications 1 à 4, dans laquelle le déclin cognitif comprend une altération de la mémoire liée à l'âge.

6. Composition à utiliser selon l'une quelconque des revendications 1 à 5, dans laquelle le sujet est un humain.

7. Composition à utiliser selon l'une quelconque des revendications 1 à 5, dans laquelle le sujet est un animal non humain.

8. Composition à utiliser selon l'une quelconque des revendications 1 à 7, dans laquelle la composition est destinée à une administration orale.

9. Composition à utiliser selon la revendication 8, dans laquelle la composition est administrée sous la forme d'une capsule ou d'un comprimé.

10. Composition à utiliser selon l'une quelconque des revendications 1 à 9, dans laquelle la composition comprend au moins 1 %, éventuellement au moins 3 %, en poids de l'extrait de *Salvia officinalis.*

11. Composition à utiliser selon l'une quelconque des revendications 1 à 10, dans laquelle la composition ne comprend pas plus de 85 % en poids de l'extrait de *Salvia officinalis.*

12. Composition à utiliser selon l'une quelconque des revendications 1 à 11, dans laquelle la composition est administrée au sujet quotidiennement, et éventuellement plus d'une fois par jour.

13. Composition à utiliser selon l'une quelconque des revendications 1 à 12, dans laquelle la composition est administrée en une quantité non inférieure à environ 1 mg du composant actif, typiquement non inférieure à environ 10 mg du composant actif, éventuellement non inférieure à environ 50 mg du composant actif, convenablement non inférieure à environ 100 mg du composant actif.

14. Composition à utiliser selon l'une quelconque des revendications 1 à 13, dans laquelle la composition est administrée en une quantité de pas plus d'environ 25 g du composant actif, convenablement pas plus d'environ 10 g, et éventuellement pas plus d'environ 1 g.

15. Composition à utiliser selon l'une quelconque des revendications 1 à 14, dans laquelle la composition est capable de provoquer une augmentation de la durée de vie moyenne des vers *C. elegans* d'au moins 10 %, convenablement d'au moins 12 %, éventuellement d'au moins 20 % par comparaison aux vers qui n'ont pas été exposés à la composition.
